# EUROPEAN PATENT SPECIFICATION

(11) **EP 3 287 037 B1**
(45) Date of publication and mention of the grant of the patent: **27.03.2019**
(21) Application number: 16202169.5
(22) Date of filing: 05.12.2016
(51) Int. Cl.: A45D 34/04, A61M 37/00

(54) **NEEDLE ARRAY-EQUIPPED COSMETICS CONTAINER**
NADEL-ARRAY-AUSGERÜSTETER KOSMETIKBEHÄLTER
RÉCIPIENT POUR PRODUITS COSMÉTIQUES ÉQUIPÉ D'UN ENSEMBLE D'AIGUILLES

(30) Priority: 23.08.2016 KR 20160106968
(43) Date of publication of application: 28.02.2018
(73) Proprietor: Han, Ki Soo, Seoul (KR)
(72) Inventor: Han, Ki Soo, Seoul (KR); Han, Na Rae, Gyeonggi-do (KR)
(74) Representative: Cabinet Chaillot

(56) References cited:
- WO-A1-2016/072060
- WO-A2-2010/085031
- KR-B1- 101 582 822

## Description

### BACKGROUND OF THE INVENTION

### 1. Field of the Invention

The present invention relates to a needle array-equipped cosmetics container, and more particularly to a needle array-equipped cosmetics container used to inject cosmetics into skin.

### 2. Description of the Related Art

Human skin is mainly composed of the epidermis, dermis, and hypodermis. In young skin, collagen and elastic fibers account for about 80% of the dermis.

However, collagen and elastic fibers in the dermis decrease in amount and contract or deform as we age. Thus, the dermis becomes thinned, the skin becomes dried or wrinkled with time, and skin elasticity breaks down.

Therefore, we usually apply to skin functional cosmetics containing nutrients such as vitamin C or peptides that are helpful in creation of collagen, to maintain elastic skin. However, it is difficult for nutrients applied to skin to penetrate through the epidermis and reach the dermis. Only a trace amount (about 0.3% of the total content) of nutrients in cosmetics can reach the dermis.

That is, the nutrients may not satisfactorily work with just a simple application thereof to the surface of the epidermis. Therefore, there is an urgent demand for development of an instrument that enables nutrients to effectively and completely reach deep inside the dermis.

WO 2016/072060 A1, WO 2010/085031 A2 and KR 101582822 B1 disclose known needle array-equipped cosmetics containers.

### SUMMARY OF THE INVENTION

Accordingly, the present invention has been made keeping in mind the above problems occurring in the related art, and the present invention is intended to propose a needle array-equipped cosmetics container that can increase a penetration rate of cosmetics into skin.

In order to accomplish the object, according to one aspect, there is provided a needle array-equipped cosmetics container including: a container unit for containing cosmetics and which is provided with a discharge hole through which the cosmetics are discharged outside; and an injection unit installed in the discharge hole of the container unit and provided with a plurality of needle arrays used to form fine holes in skin so that the cosmetics discharged through the discharge hole of the container unit can be injected into the skin through the fine holes.

Furthermore, each needle array includes a base and a plurality of needles extending from the base; and a housing member in which the needle arrays are accommodated, with the needles protruding from the housing member, and which communicates with the discharge hole of the container unit so that cosmetics can flow out to the needle array from the container unit.

The housing member includes a support portion installed in the discharge hole of the container unit and provided with a cavity for accommodating the needle arrays therein and with a communication hole formed in the cavity to enable the cavity and the discharge hole to communicate with each other; and a lid portion provided with one needle-passing hole for each needle array, hole through which the needles of one needle array pass to be exposed outside when the lid portion is put on the support portion that has the cavity in which the needle array is accommodated. In addition, the needle-passing hole of the lid portion may have a cross section area larger than that of the needle array, in terms of a longitudinal direction thereof, thereby allowing cosmetics to pass through the needle-passing hole when the needle array is accommodated in the support portion.

In addition, the needle array may have a saw-like shape in which a plurality of needles is spaced from each other and arranged in a longitudinal direction of the base.

In addition, the support portion may be assembled with the container unit in such a manner that the support portion is screwed into the discharge hole of the container, and the lid portion may be detachably fitted into the cavity of the support portion.

The needle-passing hole of the lid portion may be tapered toward an upper end of the lid portion.

In the lid portion a fluid-passing hole that allows cosmetics to pass therethrough may be arranged between the needle-passing holes.

In addition, the lid portion may have notches extending in a sideway direction from a lower end thereof and the base of the needle array has protrusions that protrude from respective sides of the base, so that the protrusions are inserted in the notches when the needles of the needle array are inserted through the needle-passing hole of the lid portion.

In addition, the injection unit may further include a sponge member that is installed on the support portion to surround a surface of the lid portion.

In addition, the needle array-equipped cosmetics container may further include a cover member assembled with the injection unit to cover the injection unit.

In addition, the needle array-equipped cosmetics container may further include an airless pump that is installed in the discharge hole of the container unit to connect the container unit with the injection unit, perform a pumping operation in response to pressing force of the injection unit, and supply cosmetics in the container unit to the injection unit.

According to the present invention, the needle array-equipped cosmetics container includes the injection unit that is equipped with the needle array used to form fine holes in skin so that cosmetics, discharged through the discharge hole of the container unit, can be injected into the skin through the fine holes formed by the needles of the needle array. Therefore, with the use of the needle array-equipped cosmetics container according to the present invention, it is possible to form fine holes in skin and thus to effectively inject a large amount of cosmetics into the skin through the fine holes.

In addition, each component of the needle array-equipped cosmetics container according to the present invention can be easily replaced and maintained because the support portion of the housing member can be detachably combined with the container unit, for example, in a screwing manner and because the lid portion also can be detachably combined with the support portion of the housing member. That is, since each component can be easily assembled with and disassembled from the other components, the needle array-equipped cosmetics container according to the present invention can be hygienically maintained and used for a long time.

In addition, the needle array-equipped cosmetics container according to the present invention is cost-effective because the injection unit can be installed in a discharge hole of any conventional container unit for cosmetics. Therefore, the cosmetics container according to the present invention can be manufactured by combining the injection unit according to the present invention with an existing container unit for cosmetics, instead of producing new container units for cosmetics. That is, any existing container unit for cosmetics, for example, a container unit equipped with an airless pump can be recycled.

### BRIEF DESCRIPTION OF THE DRAWINGS

FIG. 1 is a perspective view illustrating a needle array-equipped cosmetics container according to one embodiment of the present invention;
FIG. 2 is an exploded perspective view illustrating the needle array-equipped cosmetics container of FIG. 1;
FIG. 3 is a perspective view illustrating a needle array of the needle array-equipped cosmetics container of FIG. 2;
FIG. 4 is a perspective view illustrating an injection unit of the needle array-equipped cosmetics container of FIG. 2;
FIG. 5 is a cross-sectional view taken along line **I-I' of** FIG. 4;
FIG. 6 is a cross-sectional view taken along line II-II' of FIG. 4;
FIGS. 7A and 7B are perspective views illustrating a state in which a cosmetic material is injected into skin using the needle array-equipped cosmetics container according to one embodiment of the present invention.

### DESCRIPTION OF THE PREFERRED EMBODIMENTS

Hereinafter, exemplary embodiments of the present invention will be described with reference to the accompanying drawings. Throughout the drawings, the same reference numerals will refer to the same or like parts. In addition, in describing the present invention, descriptions of known related arts which have been deemed to obscure the gist of the present invention will be omitted below.

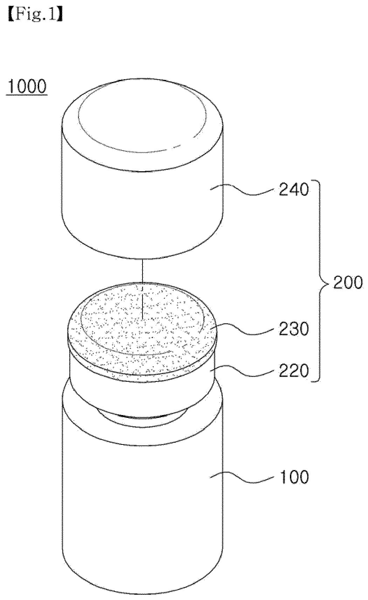

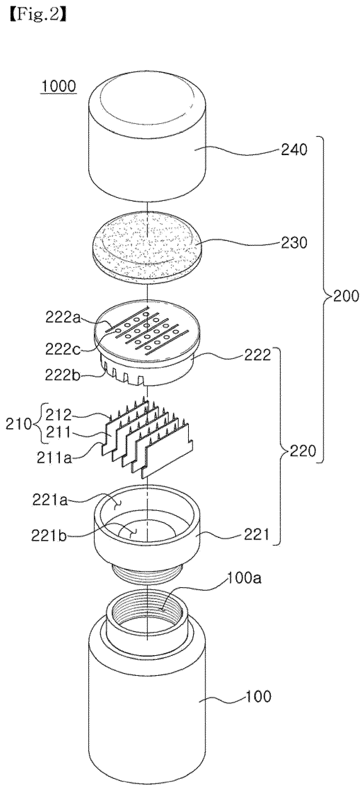

FIG. 1 is a perspective view illustrating a needle array-equipped cosmetics container according to one embodiment of the present invention, and FIG. 2 is an exploded perspective view illustrating the needle array-equipped cosmetics container of FIG. 1.

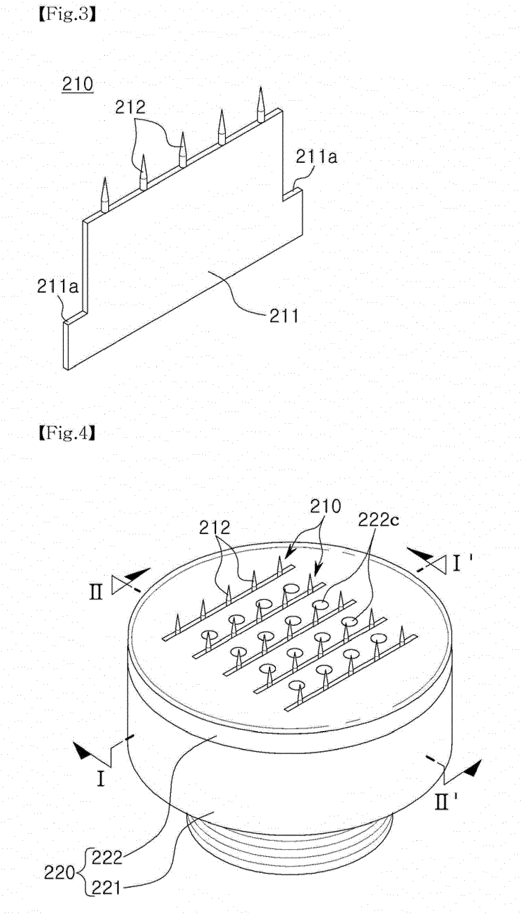

In addition, FIG. 3 is a perspective view illustrating a needle array of the needle array-equipped cosmetics container of FIG. 2, FIG. 4 is a perspective view illustrating an injection unit of the needle array-equipped cosmetics container of FIG. 2, and FIGS. 5 and 6 are cross-sectional views respectively taken along lines I-I' and II-II' of FIG. 4.

With reference to the drawings, a needle array-equipped cosmetics container 1000 according to one embodiment of the present invention includes a container unit 100 and an injection unit 200.

The container unit 100 has an internal chamber to contain cosmetics therein and a discharge hole 110a at the top of the container unit 100. The discharge hole 110a is an opening through which the cosmetics contained in the internal chamber can be discharged outside. The container unit 100 may take any form as long as it is formed to contain cosmetics therein and to have a discharge hole, meaning that the outward form of the container unit 100 is not limited to ones disclosed herein but may be similar to as conventional containers or vessels for cosmetics.

The injection unit 200 is installed in the discharge hole 100a of the container unit 100. The injection unit 200 is used to form fine holes in skin 1 with a plurality of needle arrays 210 installed therein and inject cosmetics, discharged through the discharge hole 100a of the container unit 100, into the skin 1 through the fine holes formed by needles of the needle arrays.

Therefore, with the use of the needle array-equipped cosmetics container according to the present invention 1000, it is possible to inject a large amount of cosmetics into the skin 1 while forming fine holes in the skin 1 with the needle arrays 210.

Specifically, the injection unit 200 includes a plurality of needle arrays 210 and a housing member 220.

Herein, each needle array 210 includes a base 211 and needles 212. The needles 212 extend upward from an upper end of the base 211. The needles 212 are a portion to be stuck into the skin 1.

In the present embodiment, the needle array 210 may have a saw-like shape as illustrated in FIG. 3. That is, a plurality of needles 212 is spaced from each other and arranged in a longitudinal direction of the base 211. Therefore, when the needle array 210 is put on and pressed against the skin 1, many fine holes can be simultaneously formed in the skin 1. As described below in detail, cosmetics may flow along the side surface of the base 211 and then along each needle 212, thereby finally being injected into the skin 1.

In addition, the housing member 220 is structured such that the needles 212 protrude from an upper surface of the housing member 220 when the needle arrays 210 are accommodated inside the housing member 220. In addition, the housing member 220 is structured to communicate with the discharge hole 100a of the container unit 100 so that cosmetics in the container unit 100 can flow out to the needle array 210.

Specifically, the housing member 220 includes a support portion 221 and a lid portion 222.

The support portion 221 is installed in the discharge hole 100a of the container unit 100 and provided with a cavity 221a in which the needle arrays 210 can be accommodated. The support portion 221 has a communication hole 221b in the bottom of the cavity 221a, so that the cavity 221a and the discharge hole 100a can communicate with each other through the communication hole 221b.

That is, the cavity 221a is formed in an upper portion of the support portion 221, and a plurality of needle arrays 210 are accommodated in the cavity 221a. The communication hole 221b extends from the inside of the cavity 221a to the bottom surface of the support portion 221, so that the cavity 221a of the support portion 221 can communicate with the discharge hole 100a of the container unit 100. Therefore, cosmetics contained in the container unit 100 can flow out to the cavity 221a through the discharge hole 100a and the communication hole 221b.

In addition, the lid portion 222 is fitted into the cavity 221a of the support portion 221. In this state, the needles 212 of the needle arrays 210 accommodated in the cavity 221a are inserted to pass through the needle-passing holes 222a to protrude from the upper surface of the lid portion 222.

That is, each needle-passing hole 222a is formed to extend through the lid portion 222 in a vertical direction and the needle arrays 210 are accommodated in a standing posture within the cavity 221a. In this state, each base 211 of the needle arrays 210 is put inside the cavity 221a and the needles 212 arranged at an upper end of the base 211 pass through the needle-passing hole 222a and protrude from the upper surface of the lid portion.

The needle-passing hole 222a of the lid portion 222 has a cross section area larger than that of the needle array 210, in terms of a longitudinal direction thereof so that cosmetics can flow out through the needle-passing holes 222a in a state in which the needle array 210 is installed in the cavity 221a of the support portion 221. Therefore, when the needle array-equipped cosmetics container 1000 according to the present invention is pressed against the skin 1, cosmetics contained in the container unit 100 are injected into the skin 1 through the needle-passing hole 222a of the housing member 220 and then through the fine holes in the skin formed by the needles 212 of the needle array 210. In this case, a portion of the cosmetics flows along the needles 212 of the needle array 210 when the cosmetics are injected into the skin 1 through the needle-passing hole 222a. Therefore, an event that the needles 212 produce fine holes in the skin 1 and an event that the cosmetics are injected into the skin 1 can simultaneously occur, so that an injection rate of cosmetics into the skin 1 can be increased.

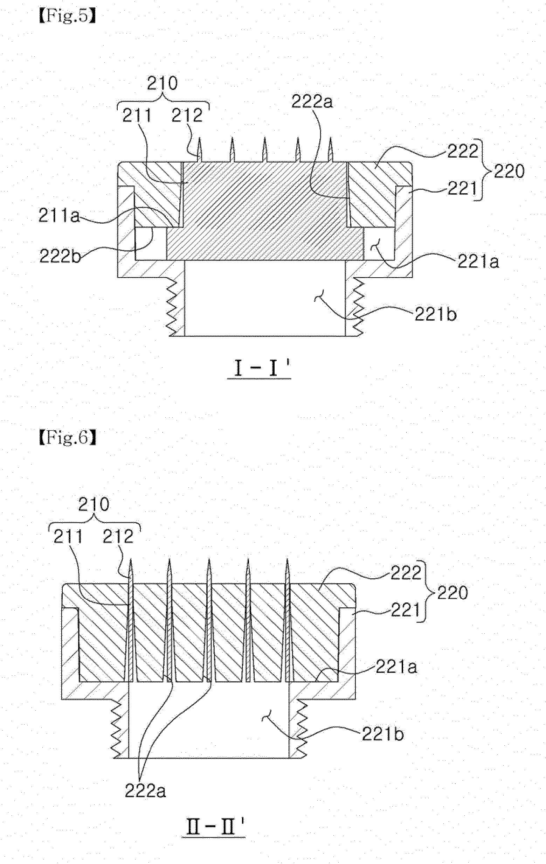

In a preferred embodiment, as illustrated in FIG. 6, the needle-passing hole 222a of the lid portion 222 is tapered toward the upper surface of the lid portion 222. That is, the size of the needle-passing hole 222a decreases from the bottom to the top of the lid portion 222. For this reason, a flow path (gap) along which cosmetics flow is formed due to a gap around the needle 212 passing through the needle-passing hole 222a, and thus the cosmetics can be supplied to the skin 1. In addition, since the gap around the needle 212 in the needle-passage hole 222a is decreased toward an upper end of the needle 212 and the needle array 210 is securely stationed without shaking even during injection of cosmetics into the skin 1, skin care treatment can be stably performed.

In the lid portion fluid-passing holes 222c, which allow cosmetics to flow therethrough, may be arranged between the needle-passing holes 222a.

The fluid-passing holes 222c increase an amount of cosmetics that flow out of the housing member 220 to be injected into the skin 1. That is, it is possible to increase an amount of cosmetics injected into the skin 1.

In addition, the lid portion 222 has a plurality of notches 222b extending in a sideway direction from a lower end thereof and the needle array 210 has protrusions 211a that protrude from the side edges of the base 211 thereof, so that the protrusions 211a interlock with the notches 222b when the needle array 210 is installed in the housing member such that the needles 212 of the needle array 210 are inserted to pass through the needle-passing holes 222a of the lid portion 222.

Since the lid portion 222 and the needle arrays 210 are provided with the notches 222b and the protrusions 211a, respectively, when the needle array 210 is installed such that the needles 212 pass through the needle-passing hole 222a of the lid portion, the protrusions 211a of the needle array 210 interlock with the notches 222b of the lid portion 222. Therefore the needles 212 of the needle array 210 that pass through the needle-passing hole 222a of the lid portion 222 do not fall apart through the needle-passing hole 222a. The needle array 210 may be securely fixed in the housing member.

In addition, the support portion 221 is detachably combined with the container unit 100 in such a manner that the support portion 221 is screwed into the discharge hole 100a of the container unit 100. The lid portion 222 is also detachably combined with the support portion 221 in such a manner that the lid portion 222 is fitted in the cavity 221a.

Therefore, when the support portion 221 is combined with the container unit 100, it can be disassembled from the container unit 100. Furthermore, the support portion 221 can be easily combined with and disassembled from the container unit 100 because they are combined in a screwing manner. In addition, since the lid portion 222 is also detachably combined with the support portion 221, replacement and maintenance of each component can be effectively and easily performed. Therefore, the needle array-equipped cosmetics container 1000 according to the present invention 1000 can be hygienically maintained and used for a long time.

Above all, since the injection unit 200 can be installed in a discharge hole of any conventional container unit for cosmetics, it is not necessary to produce additional container units for cosmetics. That is, existing container units for cosmetics can be used as the container unit used in the present invention. For example, although not illustrated in the drawings, the injection unit 200 may be combined with a conventional container unit equipped with an airless pump. Since, any conventional container unit for cosmetics can be used as the container unit of the present invention, the needle array-equipped cosmetics container 1000 according to the present invention is significantly cost-effective in terms of usability.

For reference, an airless pump will be briefly described below although it is not illustrated in the drawings. When the container unit 100 and the injection unit 200 are assembled such that the injection unit 200 is installed in the discharge hole 100a of the container unit 100, when the injection unit 200 is pressed, the airless pump performs a pumping operation so that cosmetics in the container unit 100 are supplied to the injection unit 200. Details about the internal structure of the airless pump will not be given further herein because conventional airless pumps can be used as they are in the embodiment of the present invention.

On the other hand, the injection unit 200 may further include a sponge member 230 that is mounted on the support portion 221 to cover a surface of the lid portion 222.

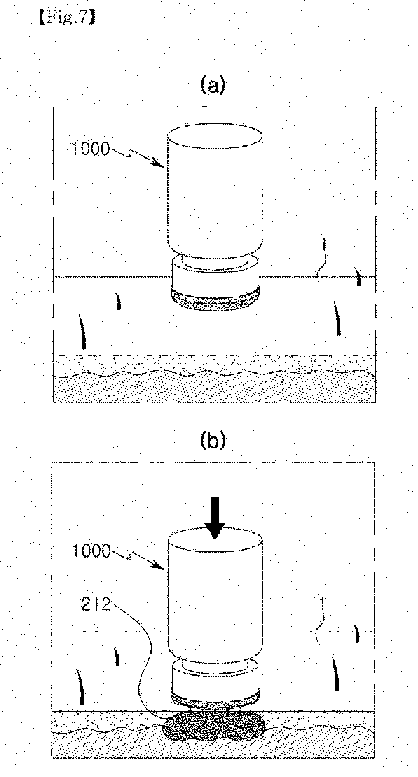

The sponge member 230 reduces feeling of irritation that a client may get when a skin care specialist presses the needle array-equipped cosmetics container 1000 according to the present invention 1000 against the client's skin 1 to inject cosmetics into the client's skin 1 as illustrated in FIG. 7.

In addition, the sponge member 230 also can retain cosmetics that are discharged through the needle-passing holes 222a and the fluid-passing holes 222c of the lid portion 220. Therefore, when the needle array-equipped cosmetics container 1000 according to the present invention is pressed against the skin 1, the sponge member 230 is contracted and thus the cosmetics retained in the sponge member 230 oozes out of the sponge member 230. Therefore, a large amount of cosmetics can be injected into the skin 1 through the fine holes formed by the needles 212 of the needle array 210.

In addition, the needle array-equipped cosmetics container 1000 according to the present invention may further include a cover member 240 that is assembled with the injection unit 200 in a manner of encasing the injection unit 200.

When the needle array-equipped cosmetics container 1000 according to the present invention is not in use, the injection unit 200 is covered by the cover member 240 to be insulated from an environment. That is, since the cover member 240 prevents the injection unit 200 from being in contact with foreign materials or impurities, the injection unit 200 can be hygienically maintained.

Next, an event in which the needle array-equipped cosmetics container 1000 according to the present invention is used to inject cosmetics into the skin 1 will be described with FIGS. 1 and 7, and particularly with FIG. 7.

When the needle array-equipped cosmetics container 1000 according to the present invention is pressed against the skin 1, the needles 212 of the needle arrays 210 are stuck into the skin 1, thereby forming fine holes in the skin 1.

In addition, when the needle array-equipped cosmetics container 1000 is pressed against the skin 1, the needle array-equipped cosmetics container 1000 is moved toward the skin 1. At this point, cosmetics are discharged outside through the discharge hole 100a of the container unit 100, and then introduced into the housing member 220. The cosmetics, having passed through the housing member 220, flow along the needles 212 of the needle arrays 210 and reach the skin 1. Finally, the cosmetics are introduced into the skin 1 through the fine holes formed therein.

In conclusion, the needle array-equipped cosmetics container 1000 according to the present invention 1000 is equipped with the injection unit 200 with which a user forms fines holes in the skin 1 and injects cosmetics, discharged through the discharge hole 100a of the container unit 100, into the skin 1 through the fine holes.

In addition, in the needle array-equipped cosmetics container 1000 according to the present invention, the support portion 221 of the housing member 220 is detachably assembled with the container unit 100, for example, in a screwing manner, so that the support portion 221 of the housing member 220 can be easily assembled with and disassembled from the container unit 100. In addition, the lid portion 222 is also detachably assembled with the support portion 221. Therefore, replacement and maintenance of each component of the needle array-equipped cosmetics container 1000 can be effectively performed. Accordingly, the needle array-equipped cosmetics container 1000 can be hygienically used for a long time.

In addition, since the injection unit 200 of the needle array-equipped cosmetics container 1000 according to the present invention can be installed in a discharge hole of any conventional container unit for cosmetics, it is not necessary to prepare additional container unit for cosmetics. That is, the injection unit 200 can be assembled with an existing container unit, for example, any cosmetics container unit equipped with an airless pump. Therefore, the needle array-equipped cosmetics container 1000 according to the present invention has high usability in terms of costs.

Although some embodiments of the present invention have been described with reference to the drawings, those skilled in the art will appreciate that the present invention is not limited to those embodiments, drawings, and disclosures but rather various modifications, additions and substitutions are possible, as long as they fall within the scope of the accompanying claims.

## Claims

1. A needle array-equipped cosmetics container (1000) comprising:
a container unit (100) for containing cosmetics therein, the container unit (100) having a discharge hole (100a) through which the cosmetics are discharged outside;
an injection unit (200) installed in the discharge hole (100a) of the container unit (100) and provided with a plurality of needle arrays (210) used to form fine holes in skin (1), thereby injecting cosmetics, discharged through the discharge hole (100a) of the container unit (100), into the skin (1) through the fine holes;
each needle array (210) including a base (211) and a plurality of needles (212) extending from the base (211); and
a housing member (220) in which the needle arrays (210) are accommodated, with the needles (212) protruding from the housing member (220), and which communicates with the discharge hole (100a) of the container unit (100) so that cosmetics flow out to the needle array (210) from the container unit (100);
the housing member (220) including:
a support portion (221) installed in the discharge hole (100a) of the container unit (100) and provided with a cavity (221a) for accommodating the needle arrays (210) therein and with a communication hole (221b) formed in the cavity (221a) to enable the cavity (221a) and the discharge hole (100a) to communicate with each other; and
a lid portion (222) provided with one needle-passing hole (222a) for each needle array, hole through which the needles (212) of one needle array (210) pass to be exposed outside when the lid portion (222) is put on the support portion (221) that has the cavity (221a) in which the needle array (210) is accommodated.

2. The needle array-equipped cosmetics container (1000) according to claim 1, wherein the needle array (210) has a saw-like shape in which the needles (212) are spaced from each other and arranged in a longitudinal direction of the base (211) .

3. The needle array-equipped cosmetics container (1000) according to claim 1, wherein the support portion (221) is detachably assembled with the container unit (100) in such a manner that the support portion (221) is screwed into the discharge hole (100a) of the container (100), and the lid portion (222) is detachably fitted into the cavity (221a) of the support portion (221).

4. The needle array-equipped cosmetics container (1000) according to claim 1, wherein the needle-passing hole (222a) of the lid portion (222) have a cross section area larger than the needle array (210) in terms of a longitudinal direction, to enable cosmetics to pass through the needle-passing hole (222a) when the needle array (210) is accommodated in the support portion (221).

5. The needle array-equipped cosmetics container (1000) according to claim 4, wherein the needle-passing hole (222a) of the lid portion (222) is tapered toward an upper surface of the lid portion (222).

6. The needle array-equipped cosmetics container (1000) according to claim 1, wherein in the lid portion (222) a fluid-passing hole (222c) that allows cosmetics to pass thererough is arranged between the needle-passing holes (222a) .

7. The needle array-equipped cosmetics container (1000) according to claim 1, wherein the lid portion (222) has notches (222b) extending in a sideway direction from a lower end thereof and the base (211) has protrusions (211a) that protrude from respective side edges of the base (211), so that the protrusions (211a) interlock with the notches (222b) when the needles (212) of the needle array (210) are inserted to pass through the needle-passing hole (222a) of the lid portion (222).

8. The needle array-equipped cosmetics container (1000) according to claim 1, wherein the injection unit (200) further includes a sponge member (230) that is mounted on the support portion (221) to surround a surface of the lid portion (222).

9. The needle array-equipped cosmetics container (1000) according to claim 1, further comprising a cover member (240) assembled with the injection unit (200) to cover the injection unit (200).

10. The needle array-equipped cosmetics container (1000) according to any one of claims 1 to 9, further comprising an airless pump installed in the discharge hole (100a) of the container unit (100) to connect the container unit (100) with the injection unit (200), perform a pumping operation in response to pressing force of the injection unit (200), and supply cosmetics in the container unit (100) to the injection unit (200).

## Patentansprüche

1. Nadel-Array-ausgerüsteter Kosmetikbehälter (1000), umfassend:
eine Behältereinheit (100), um darin Kosmetika zu enthalten, wobei die Behältereinheit (100) ein Abgabeloch (100a) aufweist, durch das die Kosmetika nach außen abgegeben werden,
eine Injektionseinheit (200), die im Abgabeloch (100a) der Behältereinheit (100) installiert ist und mit einer Vielzahl von Nadel-Arrays (210) ausgestattet ist, die verwendet werden, um feine Löcher in der Haut (1) zu bilden, wodurch Kosmetika, abgegeben durch das Abgabeloch (100a) der Behältereinheit (100) in die Haut (1) durch die feinen Löcher injiziert werden;
wobei jedes Nadel-Array (210) eine Basis (211) und eine Vielzahl von Nadeln (212) einschließt, die sich von der Basis (211) erstrecken;
und ein Gehäuseelement (220), in dem die Nadel-Arrays (210) untergebracht sind, wobei die Nadeln (212) aus dem Gehäuseelement (220) vorspringen, und das mit dem Abgabeloch (100a) der Behältereinheit (100) kommuniziert, so dass Kosmetika an das Nadel-Array (210) von der Behältereinheit (100) ausfließen;
wobei das Gehäuseelement (220) Folgendes einschließt:
einen Stützabschnitt (221), der im Abgabeloch (100a) der Behältereinheit (100) installiert ist und mit einem Hohlraum (221a), um die Nadelanordnungen (210) darin aufzunehmen, und mit einem Kommunikationsloch (221b), das im Hohlraum (221a) gebildet ist, ausgestattet ist, um dem Hohlraum (221a) und dem Abgabeloch (100a) zu ermöglichen, miteinander zu kommunizieren; und
ein Deckelabschnitt (222), der mit einem NadelDurchgangsloch (222a) für jedes Nadel-Array ausgestattet ist, einem Loch, durch das die Nadeln (212) des Nadel-Arrays (210) verlaufen, um an der Außenseite ausgesetzt zu sein, wenn der Deckelabschnitt (222) auf den Stützabschnitt (221) gestellt ist, der den Hohlraum (221a) aufweist, in dem das Nadel-Arrays (210) untergebracht ist.

2. Nadel-Array-ausgerüsteter Kosmetikbehälter (1000) nach Anspruch 1, wobei das Nadel-Array (210) eine sägeähnliche Form aufweist, in der die Nadeln (212) voneinander beabstandet und in einer Längsrichtung der Basis (211) angeordnet sind.

3. Nadel-Array-ausgerüsteter Kosmetikbehälter (1000) nach Anspruch 1, wobei der Stützabschnitt (221) lösbar derart mit der Behältereinheit (100) verbunden ist, dass der Stützabschnitt (221) in das Abgabeloch (100a) des Behälters (100) geschraubt ist, und der Deckelabschnitt (222) lösbar in den Hohlraum (221a) des Stützabschnitts (221) gepasst ist.

4. Nadel-Array-ausgerüsteter Kosmetikbehälter (1000) nach Anspruch 1, wobei das Nadeldurchgangsloch (222a) des Deckelabschnitts (222) einen Querschnittsbereich aufweist, der hinsichtlich einer Längsrichtung größer als das Nadel-Array (210) ist, um zu ermöglichen, dass Kosmetika durch des Nadeldurchgangsloch (222a) verlaufen, wenn das Nadel-Array (210) im Stützabschnitt (221) aufgenommen ist.

5. Nadel-Array-ausgerüsteter Kosmetikbehälter (1000) nach Anspruch 4, wobei das Nadeldurchgangsloch (222a) des Deckelabschnitts (222) hin zu einer oberen Fläche des Deckelabschnitts (222) verjüngt ist.

6. Nadel-Array-ausgerüsteter Kosmetikbehälter (1000) nach Anspruch 1, wobei im Deckelabschnitt (222) ein Fluiddurchgangsloch (222c), das ermöglicht, dass Kosmetika dadurch verlaufen, zwischen den Nadeldurchgangslöchern (222a) angeordnet ist.

7. Nadel-Array-ausgerüsteter Kosmetikbehälter (1000) nach Anspruch 1, wobei der Deckelabschnitt (222) Kerben (222b) aufweist, die sich in einer seitlichen Richtung von einem unteren Ende davon erstrecken, und die Basis (211) Vorsprünge (211a) aufweist, die von entsprechenden Seitenkanten der Basis (211) vorspringen, so dass die Vorsprünge (211a) mit den Kerben (222b) ineinandergreifen, wenn die Nadeln (212) des Nadel-Arrays (210) eingeführt sind, um durch das Nadeldurchgangsloch (222a) des Deckelabschnitts (222) zu verlaufen.

8. Nadel-Array-ausgerüsteter Kosmetikbehälter (1000) nach Anspruch 1, wobei die Injektionseinheit (200), weiter ein Schwammelement (230) umfasst, das auf den Stützabschnitt (221) montiert ist, um eine Fläche des Deckelabschnitts (222) zu umgeben.

9. Nadel-Array-ausgerüsteter Kosmetikbehälter (1000) nach Anspruch 1, weiter umfassend ein Abdeckelement (240), das mit der Injektionseinheit (200) zusammengebaut ist, um die Injektionseinheit (200) abzudecken.

10. Nadel-Array-ausgerüsteter Kosmetikbehälter (1000) nach einem der Ansprüche 1 bis 9, weiter umfassend eine luftlose Pumpe, die im Abgabeloch (100a) der Behältereinheit (100) installiert ist, um die Behältereinheit (100) mit der Injektionseinheit (200) zu verbinden, einen Pumpvorgang in Reaktion auf eine Druckkraft der Injektionseinheit (200) durchzuführen und Kosmetika in der Behältereinheit (100) an die Injektionseinheit (200) zu liefern.

## Revendications

1. Récipient pour produits cosmétiques équipé d'un ensemble d'aiguilles (1000) comprenant :
une unité de récipient (100) pour contenir des produits cosmétiques à l'intérieur de celle-ci, l'unité de récipient (100) ayant un trou d'évacuation (100a) à travers lequel les produits cosmétiques sont évacués à l'extérieur ;
une unité d'injection (200) installée dans le trou d'évacuation (100a) de l'unité de récipient (100) et comportant une pluralité d'ensembles d'aiguilles (210) utilisés pour former des trous fins dans la peau (1), injectant ainsi des produits cosmétiques, évacués à travers le trou d'évacuation (100a) de l'unité de récipient (100), dans la peau (1) à travers les trous fins ;
chaque ensemble d'aiguilles (210) comprenant une base (211) et une pluralité d'aiguilles (212) s'étendant à partir de la base (211) ; et
un élément de logement (220) dans lequel les ensembles d'aiguilles (210) sont reçus, avec les aiguilles (212) faisant saillie à partir de l'élément de logement (220), et qui communique avec le trou d'évacuation (100a) de l'unité de récipient (100) de telle sorte que les produits cosmétiques s'écoulent jusqu'à l'ensemble d'aiguilles (210) à partir de l'unité de récipient (100) ;
l'élément de logement (220) comprenant :
une partie de support (221) installée dans le trou d'évacuation (100a) de l'unité de récipient (100) et comportant une cavité (221a) pour recevoir les ensembles d'aiguilles (210) à l'intérieur de celle-ci et un trou de communication (221b) formé dans la cavité (221a) pour permettre à la cavité (221a) et au trou d'évacuation (100a) de communiquer l'un avec l'autre ; et
une partie couvercle (222) comportant un trou de passage d'aiguille (222a) pour chaque ensemble d'aiguilles, trou à travers lequel les aiguilles (212) d'un ensemble d'aiguilles (210) passent pour être exposées à l'extérieur lorsque la partie couvercle (222) est posée sur la partie de support (221) qui a la cavité (221a) dans laquelle l'ensemble d'aiguilles (210) est reçu.

2. Récipient pour produits cosmétiques équipé d'un ensemble d'aiguilles (1000) selon la revendication 1, dans lequel l'ensemble d'aiguilles (210) a une forme de type scie dans laquelle les aiguilles (212) sont espacées les unes des autres et disposées dans une direction longitudinale de la base (211).

3. Récipient pour produits cosmétiques équipé d'un ensemble d'aiguilles (1000) selon la revendication 1, dans lequel la partie de support (221) est assemblée de manière détachable avec l'unité de récipient (100) de telle sorte que la partie de support (221) est vissée dans le trou d'évacuation (100a) du récipient (100), et la partie couvercle (222) est montée de manière détachable dans la cavité (221a) de la partie de support (221).

4. Récipient pour produits cosmétiques équipé d'un ensemble d'aiguilles (1000) selon la revendication 1, dans lequel le trou de passage d'aiguille (222a) de la partie couvercle (122) a une section transversale plus grande que l'ensemble d'aiguilles (210) en termes de direction longitudinale, pour permettre aux produits cosmétiques de passer à travers le trou de passage d'aiguille (222a) lorsque l'ensemble d'aiguilles (210) est reçu dans la partie de support (221).

5. Récipient pour produits cosmétiques équipé d'un ensemble d'aiguilles (1000) selon la revendication 4, dans lequel le trou de passage d'aiguille (222a) de la partie couvercle (222) est effilé vers une surface supérieure de la partie couvercle (222).

6. Récipient pour produits cosmétiques équipé d'un ensemble d'aiguilles (1000) selon la revendication 1, dans lequel, dans la partie couvercle (222), un trou de passage de fluide (222c), qui permet aux produits cosmétiques de passer à travers celui-ci, est prévu entre les trous de passage d'aiguille (222a).

7. Récipient pour produits cosmétiques équipé d'un ensemble d'aiguilles (1000) selon la revendication 1, dans lequel la partie couvercle (222) a des encoches (222b) s'étendant dans une direction latérale à partir d'une extrémité inférieure de celle-ci, et la base (211) a des protubérances (211a) qui font saillie à partir de bords latéraux respectifs de la base (211), de telle sorte que les protubérances (211a) se verrouillent avec les encoches (222b) lorsque les aiguilles (212) de l'ensemble d'aiguilles (210) sont introduites pour passer à travers le trou de passage d'aiguille (222a) de la partie couvercle (222).

8. Récipient pour produits cosmétiques équipé d'un ensemble d'aiguilles (1000) selon la revendication 1, dans lequel l'unité d'injection (200) comprend en outre un élément éponge (230) qui est monté sur la partie de support (221) pour entourer une surface de la partie couvercle (222).

9. Récipient pour produits cosmétiques équipé d'un ensemble d'aiguilles (1000) selon la revendication 1, comprenant en outre un élément de recouvrement (240) assemblé avec l'unité d'injection (200) pour recouvrir l'unité d'injection (200).

10. Récipient pour produits cosmétiques équipé d'un ensemble d'aiguilles (1000) selon l'une quelconque des revendications 1 à 9, comprenant en outre une pompe à vide installée dans le trou d'évacuation (100a) de l'unité de récipient (100) pour relier l'unité de récipient (100) à l'unité d'injection (200), réaliser une opération de pompage en réponse à une force de pression de l'unité d'injection (200), et distribuer des produits cosmétiques dans l'unité de récipient (100) à l'unité d'injection (200).
